# FASCICULE DE BREVET EUROPEEN

(11) **EP 4 319 858 B1**
(45) Date de publication et mention de la délivrance du brevet: **03.06.2026**
(21) Numéro de dépôt: 22716430.8
(22) Date de dépôt: 04.04.2022
(51) Int. Cl.: A61M 37/00, C25B 1/04, C25B 9/00, C25B 9/01, C25B 9/17, C25B 15/08, A61N 1/04, A61N 1/30

(54) **DISPOSITIF DE DÉLIVRANCE TRANSDERMIQUE DE DIHYDROGÈNE**
TRANSDERMALE ABGABEVORRICHTUNG FÜR DIHYDROGEN
DIHYDROGEN TRANSDERMAL DELIVERY DEVICE

(30) Priorité: 08.04.2021 FR 2103592
(43) Date de publication de la demande: 14.02.2024
(73) Titulaire: Université Grenoble Alpes, 38400 Saint-Martin-d'Hères (FR); Centre National de la Recherche Scientifique, 75016 Paris (FR); Institut Polytechnique de Grenoble, 38000 Grenoble (FR); Institut National de la Santé et de la Recherche Médicale, 75013 Paris (FR)
(72) Inventeur: ZEBDA, Abdelkader, 38100 GRENOBLE (FR); CINQUIN, Philippe, 38330 SAINT NAZAIRE LES EYMES (FR); BEN TAHAR, Awatef, 38000 GRENOBLE (FR); COIZET, Véronique, 38240 MEYLAN (FR)
(74) Mandataire: Hautier IP
(86) Numéro de dépôt international: PCT/EP2022/058903
(87) Numéro de publication internationale: WO 2022/214439

(56) Documents cités:
- US-A1- 2020 030 598
- US-A1- 2020 345 992

## Description

### DOMAINE TECHNIQUE DE L'INVENTION

L'invention concerne le domaine de l'hydrogénothérapie. Elle trouve pour application particulièrement avantageuse le domaine de nombreuses pathologies, et notamment de pathologies inflammatoires et/ou liées au stress oxydant.

### ETAT DE LA TECHNIQUE

L'hydrogénothérapie montre un intérêt grandissant pour soigner un grand nombre de pathologies, notamment des pathologies inflammatoires et/ou liées à un stress oxydant. Une revue de plus de 300 articles liste pas moins de 166 pathologies où une délivrance de dihydrogène a été testée pour ses vertus anti-oxydantes (Ichihara, M., Sobue, S., Ito, M., Ito, M., Hirayama, M., & Ohno, K. (2015), "Beneficial biological effects and the underlying mechanisms of molecular hydrogen-comprehensive review of 321 original articles", Medical gas research, 5(1), 12).

Différentes techniques sont connues pour administrer le dihydrogène au corps humain ou animal. Le dihydrogène peut être administré sous forme de gaz à inhaler, formé par électrolyse. Cette technique est toutefois chère et incommode, car elle nécessite un appareillage complexe du type respirateur. Le dihydrogène peut être administré sous forme d'eau hydrogénée, bue par le patient.

Ces techniques montrent des limites majeures :
- une faible quantité de dihydrogène délivrée,
- de fortes variations de la concentration en dihydrogène, et
- une contrainte majeure en termes d'observance thérapeutique.

Pour s'affranchir de ces inconvénients, le dihydrogène peut être produit par électrolyse de l'eau, réalisée par un dispositif implanté dans le corps humain ou animal. Il est notamment connu du document WO 2019/122441 A1 un dispositif implantable dans le corps humain ou animal comprenant une anode et une cathode électriquement reliées à une source d'énergie. Le dispositif laisse passer un liquide corporel formant l'électrolyte, permettant de fermer le circuit électrique. Du dihydrogène est ainsi produit par électrolyse de l'eau d'un fluide corporel, à l'intérieur du corps humain. L'implantation d'un tel dispositif reste toutefois très invasive pour le patient.

Il est connu du document US 2020/0030598 A1 un dispositif de délivrance d'hydrogène, de peroxyde d'hydrogène et/ou d'oxygène par électrolyse à appliquer sur la peau. La délivrance de dihydrogène par le dispositif reste toutefois limitée.

Un objet de la présente invention est donc de proposer un dispositif de délivrance de dihydrogène peu invasif, et de préférence non invasif, pour le corps humain et animal, tout en permettant une délivrance suffisante, de préférence améliorée, de dihydrogène.

Les autres objets, caractéristiques et avantages de la présente invention apparaîtront à l'examen de la description suivante et des dessins d'accompagnement. Il est entendu que d'autres avantages peuvent être incorporés.

### RESUME DE L'INVENTION

Pour atteindre cet objectif, selon un mode de réalisation on prévoit un dispositif de délivrance de dihydrogène selon la revendication 1. Le dispositif comprend :
- un corps comprenant au moins une anode et au moins une cathode, et
- une source d'énergie électrique, l'anode et la cathode étant électriquement raccordées à la source d'énergie électrique.

Le dispositif étant configuré pour délivrer le dihydrogène à travers la peau d'un corps humain ou animal :
- le corps est à base d'un matériau souple, apte à se conformer sur la peau d'un corps humain ou animal, le corps présentant une surface extérieure comprenant :
   o une face inférieure destinée à être appliquée sur la peau et autorisant le passage de dihydrogène,
   ∘ au moins une face destinée à ne pas être en contact avec la peau,
- le corps comprend un réservoir d'eau, l'agencement relatif du réservoir, de l'anode et de la cathode étant configuré de sorte que l'eau contenue dans le réservoir est en contact avec l'anode et la cathode pour former un circuit électrique fermé, de façon à produire du dihydrogène à la cathode à partir de l'eau issue du réservoir, pour libérer de façon transdermique le dihydrogène produit.

Ainsi, le dispositif est apte à être appliqué sur la peau d'un corps humain ou animal, la délivrance transdermique permet de minimiser l'impact sur le corps par rapport aux dispositifs implantables des solutions existantes.

En outre, le réservoir d'eau comprise dans le corps et donc pouvant être considérée comme embarqué, permet une délivrance de dihydrogène indépendante d'un fluide corporel, par exemple la sueur, pour permettre une délivrance d'une quantité suffisante de dihydrogène à travers la peau du patient. Cette problématique ne se pose pas en effet pour les dispositifs implantables des solutions existantes, baignant dans un fluide corporel susceptible d'être utilisé pour produire le dihydrogène.

Le dispositif de délivrance est donc peu, et de préférence n'est pas invasif pour le corps humain ou animal, tout en permettant de délivrer une quantité suffisante de dihydrogène pour obtenir un effet bénéfique sur le corps. En fonction du contexte de l'utilisation, cette quantité peut par exemple varier de 1 µmol/heure à 40 µmol/heure. Le dihydrogène étant une molécule se diffusant rapidement, impliquant une faible rémanence à l'endroit de sa libération, le dispositif est ainsi particulièrement adapté pour la prévention et/ou le traitement de pathologies inflammatoires et/ou liées à un stress oxydant à proximité de la peau du corps humain ou animal, telles que le diabète, l'obésité, des inflammations de la peau ou des tissus l'environnant ou encore le psoriasis, un cancer de la peau, le vitiligo et toute pathologie impliquant le stress oxydatif. Le dispositif peut par ailleurs être utilisé pour la récupération musculaire, contre les courbatures par exemples.

Un deuxième aspect de l'invention concerne un article vestimentaire comprenant au moins un dispositif de délivrance de dihydrogène selon le premier aspect.

Un troisième aspect de l'invention concerne un procédé de délivrance de dihydrogène comprenant l'application sur la peau d'un corps humain ou animal, du dispositif de délivrance de dihydrogène selon le premier aspect de l'invention, et une activation du dispositif de délivrance de sorte à délivrer de façon transdermique le dihydrogène.

Comme discuté ci-dessous, selon un exemple, la délivrance transdermique de dihydrogène peut être utilisée pour la récupération musculaire, par exemple contre les courbatures. Selon un autre exemple, la délivrance transdermique de dihydrogène est utilisée pour la prévention et/ou le traitement de pathologies choisies parmi le diabète, obésité, inflammation, le psoriasis, un cancer de la peau, le vitiligo et toute pathologie impliquant le stress oxydatif.

### BREVE DESCRIPTION DES FIGURES

Les buts, objets, ainsi que les caractéristiques et avantages de l'invention ressortiront mieux de la description détaillée d'un mode de réalisation de cette dernière qui est illustré par les dessins d'accompagnement suivants dans lesquels :
La figure 1 représente une vue en coupe transversale du dispositif de délivrance selon un exemple de réalisation.
La figure 2 représente une vue en coupe transversale du dispositif de délivrance selon un autre exemple de réalisation dans lequel le dispositif comprend un matériau non perméable au dihydrogène.
Les figures 3A à 3C représentent chacune une vue en coupe transversale du dispositif de délivrance selon plusieurs exemples de réalisation, dans lesquels la face inférieure est recouverte par un matériau semi-perméable.
Les figures 4A à 4C représentent chacune une vue en coupe transversale du dispositif de délivrance selon plusieurs exemples de réalisation, dans lesquels le dispositif présente une ou plusieurs ouvertures localisées.
Les figures 5, 6A et 6B représentent chacune une vue en coupe transversale du dispositif de délivrance selon plusieurs exemples de réalisation, dans lesquels les électrodes forment un empilement.
La figure 7 représente une vue en coupe transversale du dispositif de délivrance selon un autre exemple de réalisation.
Les figures 8A et 8B représentent chacune une vue en coupe transversale du dispositif de délivrance selon plusieurs exemples de réalisation, dans lesquels les électrodes sont formées de microaiguilles destinées à pénétrer la peau.
La figure 9 représente une vue d'ensemble d'un brassard comprenant plusieurs dispositifs de délivrance de dihydrogène.

Les dessins sont donnés à titre d'exemples et ne sont pas limitatifs de l'invention. Ils constituent des représentations schématiques de principe destinées à faciliter la compréhension de l'invention et ne sont pas nécessairement à l'échelle des applications pratiques. En particulier les dimensions relatives des différents éléments composant le dispositif de délivrance ne sont pas nécessairement représentatives de la réalité.

### DESCRIPTION DÉTAILLÉE DE L'INVENTION

Avant d'entamer une revue détaillée de modes de réalisation de l'invention, sont énoncées ci-après des caractéristiques optionnelles qui peuvent éventuellement être utilisées en association ou alternativement :
- l'au moins une cathode et l'au moins une anode sont au moins en partie, et de préférence totalement, disposées dans le réservoir d'eau,
- l'au moins une cathode et l'au moins une anode sont au contact d'une paroi du réservoir,
- le corps est en partie recouvert par un matériau non perméable au dihydrogène formant au moins une portion, par exemple une première portion, et de préférence la totalité, de l'au moins une face destinée à ne pas être en contact avec la peau,
- le corps est au moins en partie recouvert par un matériau présentant un seuil de coupure inférieur à 1 µm, formant au moins une portion, par exemple une deuxième portion distincte de la première portion, de l'au moins une face destinée à ne pas être en contact avec la peau,
- la face inférieure est au moins en partie, et de préférence totalement, recouverte par un matériau semi-perméable présentant un seuil de coupure inférieur à 1 µm,
- la face inférieure est au moins en partie, et de préférence totalement, recouverte par un matériau semi-perméable présentant une perméabilité supérieure au dihydrogène par rapport à sa perméabilité au dioxygène, de préférence le matériau semi-perméable est perméable au dihydrogène et non perméable au dioxygène,
- la face inférieure est uniquement en partie recouverte par un matériau non perméable au dihydrogène de façon à former au moins une ouverture non recouverte par le matériau non perméable au dihydrogène,
- l'au moins une ouverture est recouverte par un matériau présentant une perméabilité supérieure au dihydrogène par rapport à sa perméabilité au dioxygène, de préférence le matériau semi-perméable est perméable au dihydrogène et non perméable au dioxygène,
- la face inférieure est pourvue d'au moins une, et de préférence une pluralité de, microaiguille destinée à pénétrer la peau,
- la face inférieure étant en partie recouverte par un matériau non perméable au dihydrogène de façon à former au moins une ouverture non recouverte par le matériau non perméable au dihydrogène, l'au moins une microaiguille est creuse et est disposée en regard de l'ouverture,
- le réservoir d'eau comprend, de préférence est sous la forme de, un hydrogel. Selon un exemple, l'hydrogel est composé au moins à 95 % en masse, et de préférence au moins à 99% en masse d'eau,
- la cathode et l'anode sont disposées en regard de la face inférieure destinée à être en contact avec la peau,
- la cathode et l'anode forment un empilement, la cathode étant en regard de la face inférieure destinée à être en contact avec la peau, et l'anode étant en regard d'une face destinée à ne pas être en contact avec la peau,
- au moins l'une parmi la cathode et l'anode s'étendent transversalement dans le réservoir de façon à diviser le réservoir en deux parties distinctes, sans circulation d'eau entre les deux parties. Selon un exemple, une première partie communique avec la face inférieure, et une deuxième partie communique avec une face destinée à ne pas être en contact avec la peau,
- la face inférieure étant pourvue d'au moins une, et de préférence une pluralité de, microaiguille destinée à pénétrer la peau, la cathode est formée par ladite microaiguille. Selon un exemple, la cathode et l'anode sont chacune formées par une microaiguille.

Dans la suite de la description, le terme « sur » ne signifie pas nécessairement « directement sur ». Ainsi, lorsque l'on indique qu'une pièce ou qu'un organe A est en appui « sur » une pièce ou un organe B, cela ne signifie pas que les pièces ou organes A et B soient nécessairement en contact direct avec l'autre. Ces pièces ou organes A et B peuvent être soit en contact direct soit être en appui l'une sur l'autre par l'intermédiaire d'une ou plusieurs autres pièces.

Dans la description détaillée qui suit, il pourra être fait usage de termes tels que « transversal », « supérieur », « inférieur », « intérieur », « extérieur ». Ces termes doivent être interprétés de façon relative en relation avec la position normale d'utilisation dispositif de délivrance, une fois appliqué sur la peau. Par exemple, la notion de « inférieur » correspond aux faces ou éléments en regard et/ou au contact de la peau du corps humain ou animal sur lequel le dispositif est destiné à être appliqué. La notion de « supérieur » correspond aux faces ou éléments tournés à l'opposé de la peau du corps humain ou animal sur lequel le dispositif est destiné à être appliqué.

On entend par un paramètre « sensiblement égal/supérieur/inférieur à » une valeur donnée, que ce paramètre est égal/supérieur/inférieur à la valeur donnée, à plus ou moins 10 % près, voire à plus ou moins 5 % près, de cette valeur.

On entend par un élément « à base » d'un matériau A, un élément comprenant ce matériau A et éventuellement d'autres matériaux, par exemple des additifs.

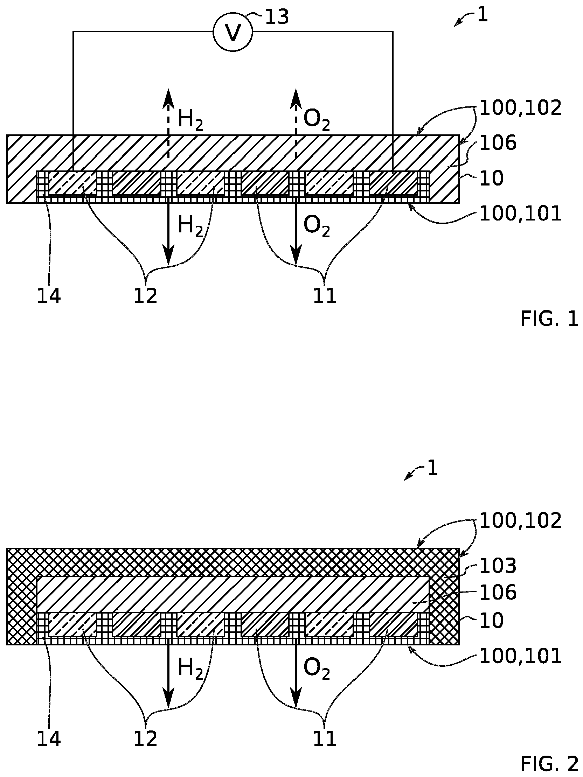

Le dispositif 1 de délivrance est maintenant décrit selon plusieurs exemples de réalisation illustrés par les figures 1 à 9.

Comme illustré par la figure 1, le dispositif 1 comprend un corps 10. Le corps 10 comprend des électrodes : au moins une anode 11 et au moins une cathode 12, pour la production de dihydrogène par électrolyse de l'eau. Pour cela, les électrodes 11, 12 sont reliées à une source d'énergie électrique 13. La source d'énergie électrique 13 n'est représentée que dans la figure 1 afin de ne pas encombrer les autres figures.

Le corps 10 comprend en outre un réservoir d'eau 14, de façon à fournir l'eau nécessaire à la fermeture du circuit électrique avec les électrodes 11, 12 et à la production de dihydrogène par électrolyse. De façon connue pour l'homme du métier, les électrodes 11, 12, la source d'énergie électrique 13 et l'eau du réservoir 14 formant un circuit électrique fermé, la source d'énergie électrique 13 alimente le circuit et impose une tension suffisante pour induire l'électrolyse de l'eau, avec la réduction de l'eau à la cathode pour former le dihydrogène, et l'oxydation de l'eau à l'anode pour former du dioxygène, lors du fonctionnement du dispositif.

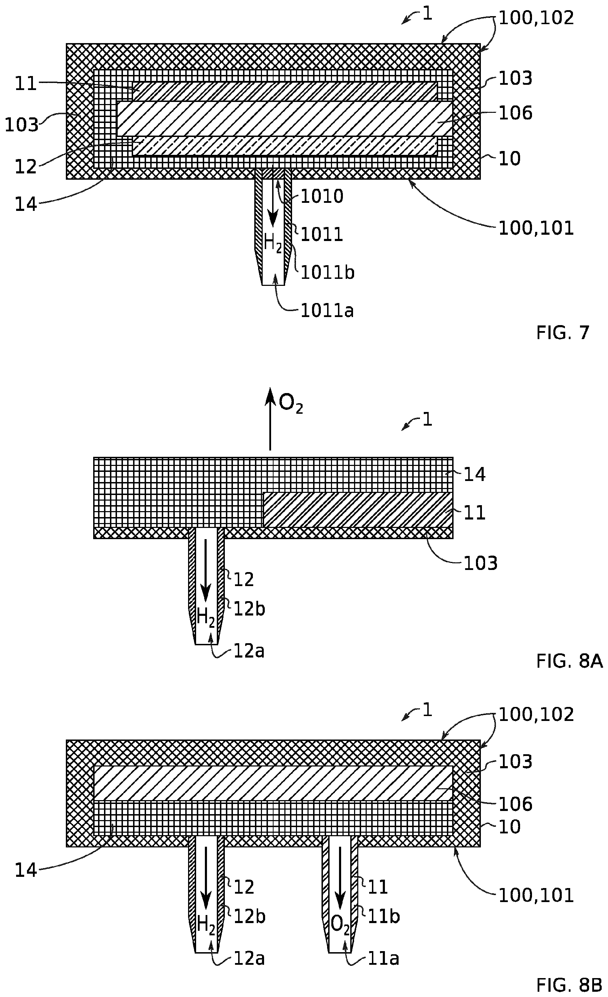

L'agencement relatif du réservoir 14, de l'anode 11 et de la cathode 12 est configuré de sorte que l'eau contenue dans le réservoir 14 est en contact avec l'anode 11 et la cathode 12 pour former un circuit électrique fermé. Selon un exemple illustré en figure 1, l'anode et la cathode peuvent pénétrer en partie, voire être totalement inclues dans le réservoir 14. Selon un autre exemple illustré en figure 8A et 8B, l'anode et/ou la cathode peuvent être directement en contact avec une paroi du réservoir 14. Le réservoir 14 peut être délimité ou formé d'un matériau laissant passer l'eau, de sorte que les électrodes 11, 12 en contact avec le réservoir d'eau sont en contact avec l'eau.

Le corps 10 est apte à se conformer sur un tissu externe du corps humain ou animal 3, par exemple un épithélium, pour permettre une délivrance transdermique du dihydrogène. Par tissu externe, on entend que le tissu forme une interface entre le corps humain et son environnement extérieur, comme par exemple la peau ou la cornée. Le corps 10 est apte à se conformer sur la peau d'un corps humain ou animal 3. Le corps 10 est configuré pour présente une souplesse suffisante pour suivre les contours du corps humain ou animal sur lequel il est appliqué. Le corps est à base d'un ou plusieurs matériaux souples. De préférence, le corps 10 comprend au moins un matériau flexible, tel que le polyéthylène téréphtalate, ou un élastomère et plus particulièrement un fluoroélastomère ou un perfluoroélastomère (par exemple le Viton^{™} commercialisé par la société Chemours, ou le Tecnoflon^{®} commercialisé par la société Solvay). La surface externe 100 du corps 10 présente ainsi une face inférieure 101 destinée à être appliquée sur la peau, à travers laquelle la délivrance de dihydrogène a lieu. Le dispositif 1 permet ainsi une délivrance transdermique de dihydrogène, sans nécessiter d'opération invasive telle que l'implantation d'un dispositif d'électrolyse dans le corps. De préférence, la face inférieure 101 est destinée à être totalement en contact avec la peau. La surface externe 100 du corps 10 présente en outre au moins une face 102 non destinée à être appliquée sur la peau, par exemple une face supérieure et des faces latérales.

Lors du développement de l'invention, il a en effet été mis en évidence que pour certaines pathologies ou traumatismes, et notamment ceux touchant la peau ou les tissus environnants tels que les tissus graisseux ou les muscles, une délivrance de dihydrogène par voie cutanée est suffisante pour soulager et/ou traiter la pathologie ou le traumatisme. Afin de garantir la délivrance d'une quantité suffisante de dihydrogène, le réservoir 14 d'eau permet de s'affranchir de l'électrolyse d'un fluide corporel, et plus particulièrement la sueur.

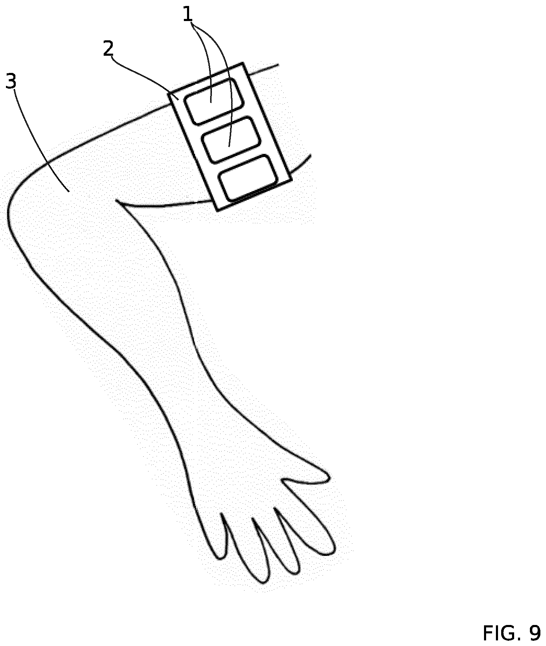

Ainsi le dispositif peut se présenter sous la forme d'un patch à appliquer sur la peau, s'étendant principalement dans un plan comme illustré par les figures 1 à 8B. Selon un exemple alternatif ou complémentaire, un ou plusieurs dispositifs de délivrance 1 peuvent être compris dans un article vestimentaire 2 destiné à être porté par un utilisateur. L'article vestimentaire peut se présenter sous la forme d'un brassard ou d'un ruban pour entourer par exemple le poignet, le bras, la jambe, le pied, ou le front de l'utilisateur. Selon l'exemple illustré en figure 9, l'article 2 peut être un brassard comprenant une pluralité de dispositifs 1, destiné à être porté sur le corps 3 et plus particulièrement autour du bras d'un utilisateur. L'article vestimentaire peut se présenter sous la forme d'un vêtement, tel qu'un t-shirt ou un haut, ou d'un sous-vêtement, de préférence configuré pour mouler la partie du corps 3 à traiter, ou encore.

Le dispositif 1 s'étendant principalement dans un plan afin de limiter de gêner les mouvements de l'utilisateur. De préférence, le dispositif présente dans ce plan des dimensions comprises entre le centimètre (cm) et plusieurs dizaines de centimètres. Selon une direction perpendiculaire à ce plan, le dispositif présente de préférence une dimension inférieure à 5 cm, de préférence inférieure à 2 cm, et plus préférentiellement encore inférieure à 1 cm. Ces dimensions s'entendent avec la source d'énergie 13 lorsqu'elle est intégrée au corps 10, ou sans la source d'énergie 13 lorsque celle-ci est déportée.

Le dispositif 1 peut être utilisé dans un procédé de délivrance de dihydrogène. Le procédé de délivrance (qui ne fait pas partie de cette invention) comprend l'application sur la peau d'un corps humain ou animal. du dispositif 1, et son activation de sorte à délivrer de façon transdermique le dihydrogène. Le procédé peut en outre comprendre le raccordement électrique du dispositif à la source d'énergie électrique 13, notamment lorsqu'elle déportée du corps 10. Le procédé peut aussi comprendre la commande de la fermeture et l'ouverture du circuit électrique formé par des moyens prévus à cet effet.

Par animal au sens de l'invention, on peut notamment entendre les grands animaux tels que les bovins, les animaux de sport tel que le cheval, les animaux de compagnie comme les chiens et les chats, et les animaux de laboratoires tels que les rats, les souris et les singes.

Le dispositif 1 est maintenant décrit en détail relativement aux différents éléments qui le composent.

Le réservoir 14 est configuré, avec les électrodes 11, 12, de sorte que les électrodes soient en contact avec l'eau du réservoir pour former le circuit électrique fermé. Le réservoir peut être sous la forme d'un réservoir creux délimité par un matériau et contenant de l'eau. Le matériau délimitant ce matériau peut être perméable à l'eau, notamment lorsque les électrodes 11, 12 sont disposées en contact avec le réservoir, de sorte que les électrodes 11, 12 sont en contact avec de l'eau issue du réservoir 14 pour produire le dihydrogène.

Selon un exemple alternatif ou complémentaire, le réservoir peut comprendre, de préférence être formé de, un matériau poreux dans lequel l'eau est contenue. Par exemple, le matériau poreux peut être une éponge ou un tissu. Le matériau poreux est de préférence à base d'un ou plusieurs polymères, tel que le polyacrylamide (PAAm), le poly(p-phenyl-p-phthalamide), des nanofibres d'aramide, de chitosane, ou l'alcool polyvinylique (PVA). Selon un exemple préférentiel, le réservoir 14 comprend un hydrogel. Selon une possibilité, le réservoir 14 est formé par l'hydrogel, le réservoir est constitué de l'hydrogel. Par hydrogel, on entend un gel dans lequel l'agent gonflant est l'eau. La matrice d'un hydrogel est généralement un réseau d'un ou plusieurs polymères. Un hydrogel présente l'avantage d'être souple et de présenter une grande capacité à contenir de l'eau. L'hydrogel peut être composé au moins à 95%, et de préférence au moins à 99% d'eau, de façon à maximiser la quantité d'eau contenue dans le réservoir 14.

De préférence, le réservoir 14 présente une hauteur sensiblement comprise entre 0,5 cm et 5 cm. La hauteur est prise selon une direction perpendiculaire à la face du corps 10 destinée à être appliquée sur la peau. La hauteur du réservoir participe à la définition du volume d'eau disponible pour électrolyse et donc de la durée d'utilisation du dispositif. La hauteur est aussi un paramètre important pour la praticité de l'utilisation et l'aspect esthétique lors du port du dispositif. Le choix de la hauteur du réservoir pourra donc être fait selon l'application visée et l'endroit du corps sur lequel est destiné à être appliqué le dispositif.

L'eau du réservoir 14 forme un électrolyte suffisamment conducteur pour permettre l'électrolyse de l'eau aux électrodes 11, 12. De façon connue pour l'homme du métier, cet électrolyte contient pour cela des sels comprenant des cations et des anions tels que Na⁺, K⁺, Ca²⁺, Cl⁻ et HCO³⁻.

Le réservoir d'eau 14 peut être rechargeable, par exemple par le biais d'une injection d'eau par une seringue dans le réservoir 14. Pour cela, le dispositif 14 peut comprendre une ouverture étanche, non représentée sur les figures, permettant une communication entre le réservoir 14 et la surface externe 100 du corps 10. L'ouverture étanche comprend par exemple un joint d'étanchéité au travers duquel une seringue peut injecter de l'eau pour recharger le réservoir 14. Selon un exemple alternatif ou complémentaire, le réservoir d'eau 14 peut être monté amovible dans le dispositif 1 pour être remplaçable. Selon un autre exemple, le corps 10 du dispositif 1 peut être un consommable destiné à être changé, notamment une fois que l'eau du réservoir 14 est utilisée.

Le corps 10 du dispositif 1 peut comprendre un élément structurel interne 106 donnant structure au corps 10 et lui conférant sa souplesse. De préférence, le corps 10 du dispositif est déformable sous la pression d'un doigt d'un utilisateur.

Comme illustré dans les figures 1 à 7, le corps 10 est configuré pour recevoir le réservoir 14 dans une forme en creux. Cette forme peut par exemple être formée par l'élément structurel interne 106, éventuellement en association avec d'autres éléments du corps 10. La forme en creux est de préférence en regard de, c'est-à-dire tournée vers, la face inférieure 101, dans laquelle le réservoir 14 est placé. Comme illustré en figure 1, l'élément structurel interne 106 ne fait pas nécessairement barrière aux gaz produits par électrolyse.

L'élément structurel interne 106, et les éventuels matériaux formant la surface externe 100 du corps, décrits ultérieurement, sont de préférence électriquement isolants. Selon un exemple, l'élément structurel interne 106 est à base ou fait de polymère tels que du polyéthylène téréphtalate (PET), du poly(methylmethacrylate) (PMMA), du polyamide, du graphène, une résine photosensible de type résine SU-8, du polyester, de la cellulose (par exemple un papier de transfert de tatouage).

La source d'énergie électrique 13 peut être intégrée au corps 10 ou être déportée, c'est à dire disposée à distance du corps 10 du dispositif 1 et reliée au corps 10 par des connexions électriques. La source d'énergie peut être :
- une pile, de préférence une pile à haute densité d'énergie, par exemple une pile au lithium,
- un dispositif de récupération d'énergie mécanique, exploitant par exemple l'effet piézoélectrique,
- une biopile capable de produire de l'électricité en consommant des espèces chimiques, typiquement naturellement présentes dans l'organisme humain ou animal, telles que : glucose, glucides, lipides, protéines,
- un dispositif de récupération d'énergie solaire, par exemple un module photovoltaïque ou une cellule Grätzel,
- un dispositif de récupération d'énergie thermique, par exemple un module thermoélectrique exploitant l'effet Seebeck.

La source d'énergie 13 est de préférence capable de produire un voltage sensiblement inférieur ou égal à 1,4 V, afin d'éviter la formation de Cl₂ à partir des ions de Cl⁻. La source d'énergie 13 peut être capable de produire un voltage sensiblement supérieur à 1,4 V, afin d'augmenter la puissance du dispositif 1 et donc la quantité d'hydrogène délivrée. Pour éviter une libération de Cl₂ à la peau, l'électrolyte dans le réservoir 14 peut être exempt d'ions Cl⁻. En alternative ou en complément, une membrane peut être configurée pour empêcher le passage du Cl₂ produit, par exemple une membrane entourant le réservoir 14, ou bien une membrane sur la face inférieure 101 en contact avec la peau, comme décrit ultérieurement. On peut adjoindre à la source d'énergie 14 un réducteur ou un élévateur de tension. Sauf mention contraire dans ce qui suit, lorsque l'on parle de ce que fournit la source d'énergie, c'est ce qu'elle fournit seule ou en association avec le réducteur ou l'élévateur de tension. La puissance pour produire une micromole de H₂/heure est sensiblement égale ou supérieure à 60µW.

Le dispositif peut comprendre en outre un réducteur de tension qui permet d'obtenir un voltage d'alimentation de l'électrolyseur sensiblement inférieur ou égal à 1,4 V. Ceci est particulièrement utile lorsque la source d'alimentation 14 produit un voltage supérieur à 1,4 V. Lorsqu'il y a plusieurs dispositifs 1 ou plusieurs paires de cathodes 12 et d'anodes 11, la source d'énergie 14 peut être propre à chacun(e) ou partagée.

Selon un exemple, la surface externe 100 est en partie recouverte par, ou de façon équivalente formée par, un matériau non perméable 103 au dihydrogène 103 formant au moins une portion 1020, et de préférence la totalité, des faces 102 du corps 10 destinées à ne pas être en contact avec la peau. Ainsi, la diffusion du dihydrogène, voire en outre celle du dioxygène, peut être contrainte dans un plan ou selon une direction préférentielle, et notamment vers la peau, comme illustré par la figure 2. Les molécules gazeuses se diffusent particulièrement rapidement et en trois dimensions par rapport à d'autres principes actifs. Contraindre leur diffusion permet de limiter la perte en principe actif lors de la délivrance, et donc d'améliorer la délivrance du dihydrogène. Cette problématique ne se pose pas dans les solutions existantes de dispositifs implantés, le dihydrogène étant alors délivré au corps quelle que soit sa direction de délivrance. Notons que si le matériau 103 est non perméable au dihydrogène, le matériau ne sera pas non plus perméable au dioxygène.

Le matériau non perméable au dihydrogène 103 est de préférence à base ou fait d'un ou plusieurs polymères choisi parmi :
- les thermoplastiques semi-cristallins, tels que le polyéthylène, le polyamide, le polychlorotrifluoroéthylène, le polyetheretherketone (PEEK), le polypropylène, le polychlorure de vinyle surchloré (CPVC), le polychlorure de vinyl (PVC), et leurs dérivés,
- les élastomères, tels que le polybutadiène, le polychloroprène (par exemple le Neoprene commercialisé par la société Nemours), les terpolymères d'éthylène et de propylène, les copolymères d'éthylène et de propylène, le poly(butadiène-coacrylonitrile) hydrogéné, le poly(isobutylène-co-isoprène), caoutchoucs de silicone avec différents substituants sur la chaîne polymère (par exemple phényle, vinyle, et/ou méthyle), caoutchouc nitrile, les fluoroélastomères, ou les perfluoroélastomères par exemple :
   ∘ de fluorure de vinylidène et au moins un parmi de l'hexafluoropropylène, du tétrafluoroéthylène, un éther vinylique fluoré, du propylène, de l'éthylène,
   o les fluoroélastomère de type Viton^{™} (polymère commercialisé par la société Chemours), les perfluoroélastomères de typeTecnoflon^{®} (polymère commercialisé par la société Solvay),
   ∘ les caoutchoucs de silicone ayant des groupes substituants fluorés sur la chaîne polymère (caoutchouc de fluorosilicone),

De préférence, sensiblement au moins 50%, de préférence au moins 70%, plus préférentiellement au moins 90 % et plus préférentiellement encore la totalité des faces 102 non destinées à être appliquées sur la peau est formée par le matériau non perméable au dihydrogène 103. Plus la portion des faces 102 formée par le matériau non perméable au dihydrogène 103 est étendue, meilleur est le ciblage du dihydrogène produit, et donc plus grande est la quantité de dihydrogène délivrée à la peau.

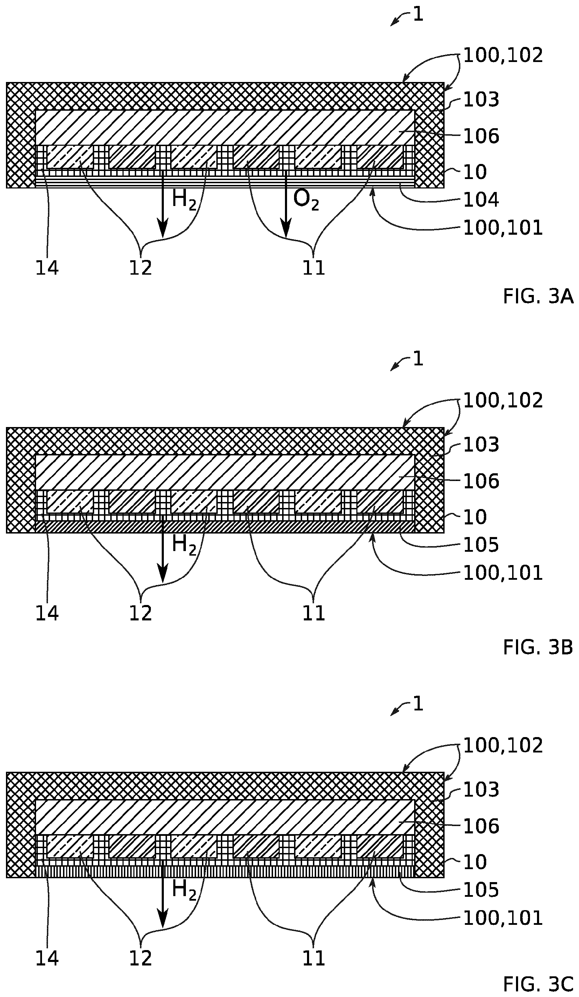

Selon un exemple, la face inférieure 101 du corps peut être recouverte ou de façon équivalente formée par un matériau dont le seuil de coupure permet de moduler les espèces susceptibles de passer par cette face 101 du dispositif 1 à la peau et inversement. Selon un exemple illustré en figure 3A, la face inférieure 101 peut être au moins en partie formée par un matériau semi-perméable 104 présentant un seuil de coupure inférieur à 1 µm. Ainsi, ce matériau 104 forme une barrière antimicrobienne empêchant d'éventuelles bactéries et/ou microorganismes de la peau de rentrer dans le dispositif 1, et plus particulièrement dans le réservoir 14, tout en permettant le passage du dihydrogène et du dioxygène. En effet, l'électrolyse de l'eau induisant la production de dioxygène, le réservoir 14 est un milieu propice à la croissance microbienne.

Comme illustré par exemple en figure 3B, la face inférieure 101 est au moins en partie formée par un matériau semi-perméable 105 configuré pour favoriser le passage du dihydrogène par rapport au dioxygène. Le matériau semi-perméable 105 présente une perméabilité au dihydrogène supérieure à sa perméabilité au dioxygène. Selon un exemple plus particulier, le matériau semi-perméable 105 est non perméable à l'oxygène et est perméable à l'hydrogène. Pour cela, la perméabilité d'un matériau à un gaz, et notamment d'un matériau polymère, dépend principalement, de façon connue par l'homme du métier, du seuil de coupure du matériau, de la solubilité du gaz dans le matériau et du coefficient de diffusion du gaz dans le matériau. Par exemple, le matériau semi-perméable 105 moins perméable et de préférence non perméable à l'oxygène, et perméable à l'hydrogène peut être un matériau avec une perméabilité à l'oxygène inférieure ou égale à 10⁻¹⁷ mol/(m.s.Pa) et une perméabilité à l'hydrogène supérieure ou égale 10⁻¹⁴ mol/(m.s.Pa).

L'homme du métier saura choisir le matériau apte à cette fin parmi les matériaux existants dans le domaine, et par exemple le caoutchouc, une résine photosensible de type résine SU-8, du polyisobutylène tel que le Vistanex^{™} (fabriqué par ExxonMobil Chemical). Ainsi, en plus de former une barrière antimicrobienne, le matériau semi-perméable empêche le passage du dioxygène produit à l'anode à travers la face inférieure 101 du corps 10. Seul le dihydrogène est délivré à la peau, ce qui permet de limiter une réaction entre le dihydrogène et le dioxygène dans le corps du patient et ainsi d'augmenter la quantité de dihydrogène délivré. Par ailleurs, de façon synergique avec le recouvrement des faces 102 avec un matériau non perméable au dihydrogène 103, le dioxygène peut se retrouver piégé dans le réservoir 14, ce qui induit une augmentation de la pression dans le réservoir 14 et favorise le passage du dihydrogène à travers la face inférieure 101. Selon un exemple, le matériau 105 présente un seuil de coupure inférieur à 32 Da.

Le matériau semi-perméable 105 peut être une membrane semi-perméable, par exemple à base ou faite d'au moins un polymère choisi parmi le poly(éthylène téréphtalate), et le polycarbonate, selon l'exemple illustré en figure 3B. Le matériau semi-perméable 105 peut être une couche formée par un matériau poreux, par exemple à base ou faite d'au moins un polymère choisi parmi le poly(éthylène téréphtalate), et le polycarbonate, selon l'exemple illustré en figure 3C.

Selon un exemple, la face inférieure 101 peut être en partie recouverte par le matériau non perméable au dihydrogène 103 de façon à former au moins une ouverture 1010 localisée de passage du dihydrogène. Ainsi, le dihydrogène n'est délivré à la peau qu'au niveau de cette ou ces ouverture(s) 1010, améliorant ainsi le ciblage du dihydrogène à la peau. De préférence, chaque ouverture présente une surface sensiblement inférieure à 1/5^{ème}, de préférence à 1/10^{ème} de la surface totale de la face inférieure 101.

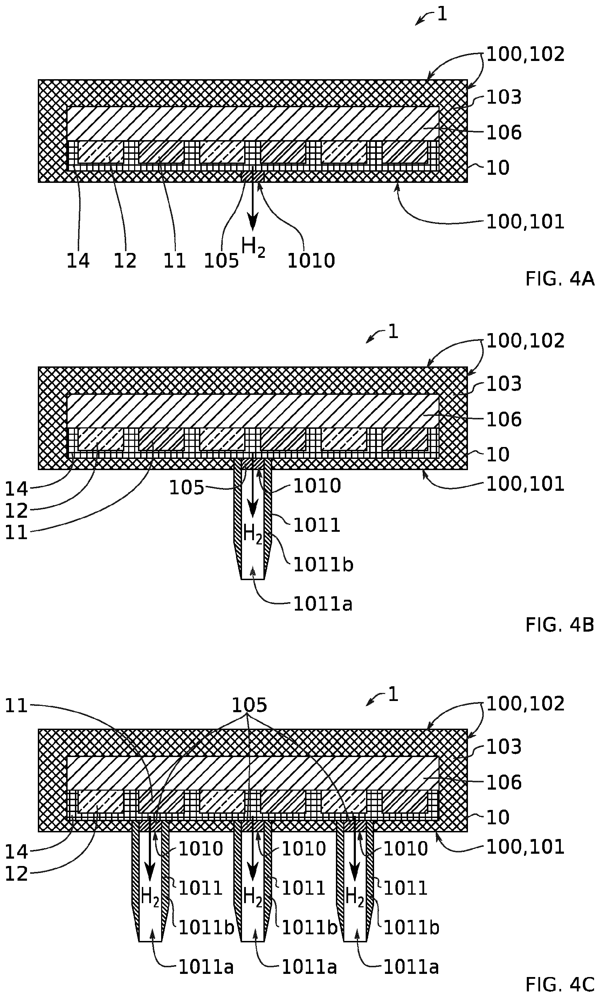

Comme illustré par exemples par les figures 4A à 4C, chacune de ces ouvertures 1010 peut être recouverte par un des matériaux 104, 105 décrits précédemment, dont le seuil de coupure permettra alors de moduler les espèces susceptibles de passer par cette ouverture 1010 du dispositif 1 à la peau et inversement. Selon les exemples illustrés, l'ouverture 1010 est recouverte par un matériau semi-perméable 105 présentant une perméabilité supérieure au dihydrogène par rapport à sa perméabilité au dioxygène, préférentiellement perméable au dihydrogène et non perméable au dioxygène, tel que précédemment décrit, pour favoriser la délivrance du dihydrogène.

En complément à cette ouverture, une microaiguille 1011 creuse peut être disposée au niveau de l'ouverture 1010. La microaiguille 1011 peut comprendre un micro-canal ou âme creuse débouchant de part et d'autre de la microaiguille, selon sa direction principale d'extension. Cette microaiguille 1011 est destinée à pénétrer la peau, comme illustré par exemple par la figure 4B. La microaiguille peut comprendre une paroi extérieure 1011b et une âme centrale creuse 1011a, la paroi extérieure 1011b étant disposée de part et d'autre de l'ouverture localisée, et l'âme centrale creuse 1011a communiquant avec l'ouverture 1010. Ainsi la microaiguille 1011 permet de faciliter la mise en contact de la face inférieure 101 du corps 10 sur la peau, et limiter le risque de décrochage du dispositif 1. En outre, la microaiguille 1011 étant creuse, elle permet de faciliter le passage du dihydrogène à travers la surface de la peau. La microaiguille comprend un micro-canal débouchant assurant le passage du dihydrogène depuis le corps 10 jusqu'à la peau et préférentiellement au moins au travers des couches supérieures de l'épiderme. La face inférieure 101 peut présenter une pluralité d'ouvertures 1010, par exemple formant un réseau d'ouvertures, chacune pouvant être dotée d'une microaiguille 1011.

On peut prévoir en alternative ou en complément, que la face inférieure 101 présente au moins une, et de préférence une pluralité de microaiguilles pleines ou creuses et destinées à pénétrer la peau, non disposées au niveau d'une ouverture 1010, par exemple la face inférieure 101 ne présentant pas d'ouverture. Ainsi les microaiguilles facilitent la mise en contact de la face inférieure 101 du corps 10 sur la peau, et limitent le risque de décrochage du dispositif 1.

Les microaiguilles précédemment décrites peuvent être à base ou faites d'un matériau rigide et biocompatible. Le matériau rigide et biocompatible peut être un métal (par exemple en titane, nickel, un alliage nickel fer, or, platine ou acier inoxydable) ou un polymère rigide, tel que l'acide polylactique, la carboxyméthylcellulose, l'acide polyglycolique, le polyvinylpyrrolidone, l'acide glycolique polylactique. Les microaiguilles sont de préférence de taille micrométrique, de préférence de longueur inférieure 1,000 µm, et de préférence de largeur ou de diamètre inférieur à 500µm.

En alternative ou en complément, pour favoriser le maintien du corps 10 du dispositif 1 au contact de la peau, on peut prévoir qu'au moins une portion de la face inférieure 101 comprenne une couche adhésive, par exemple une colle. En alternative ou en complément, pour favoriser le maintien du corps 10 du dispositif 1 en contact de la peau, on peut prévoir que l'article vestimentaire 2 ou que le dispositif 1 comprenne un support de maintien configuré pour maintenir en contact le corps 10 avec la peau.

La cathode 12 et l'anode 11 peuvent être disposées en regard de la face inférieure 101 destinée à être en contact avec la peau. Les électrodes peuvent être disposées dans un même plan sensiblement parallèle à la face inférieure 101. Ainsi, la délivrance de l'ensemble des gaz produit par électrolyse à la peau est favorisée, notamment lorsque les électrodes sont disposées sur l'élément structurel interne 106. Selon un premier exemple illustré par les figures 1 à 4C, les électrodes 11, 12 peuvent être disposées sur une même face de l'élément structurel interne 106, de préférence en regard de la face inférieure 101 et donc tournées vers la peau. Les électrodes 11, 12 sont alors disposées en vis-à-vis de la face inférieure 101. Ainsi, la diffusion des gaz produits vers la face inférieure 101, et donc vers la peau, est favorisée.

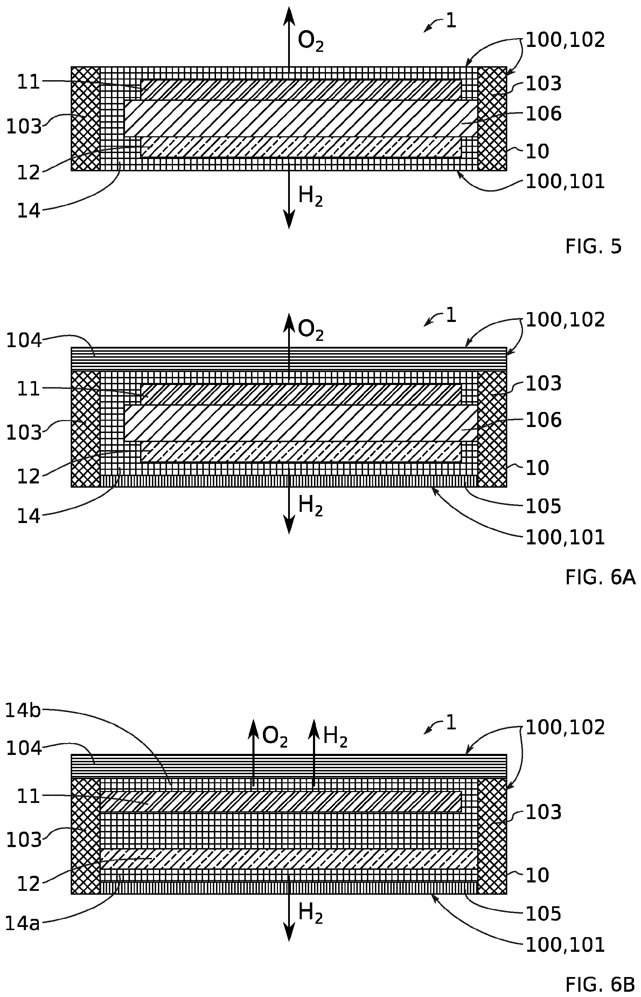

Selon un deuxième exemple, illustré par les figures 5 à 7, la cathode 12 et l'anode 11 forment un empilement. Par empilement, on entend que les électrodes sont au moins en partie juxtaposées selon une direction perpendiculaire à la face 101 destinée à être en contact avec la peau, sans nécessairement être directement en contact l'une de l'autre. La cathode 12 est de préférence en regard de la face inférieure 101, et l'anode 11 étant en regard d'une face 102 destinée à ne pas être en contact avec la peau, par exemple de la face 102 supérieure. La cathode 12 est ainsi tournée vers la face inférieure 101, et l'anode 11 est tournée vers une face 102 destinée à ne pas être en contact avec la peau, par exemple de la face 102 supérieure. Ainsi, le dioxygène est produit à l'anode en regard d'une face 102 destinée à ne pas être en contact avec la peau, et le dihydrogène est produit à proximité de la face inférieure. La délivrance du dihydrogène à la peau est favorisée, par rapport à celle du dioxygène, pour limiter une éventuelle réaction entre ces deux gaz et ainsi augmenter la quantité de dihydrogène délivrée.

Selon un exemple, illustré dans les figures 5, 6A et 7, l'anode 11 peut être disposée sur une face de l'élément structurel interne 106 et la cathode sur une face opposée de cet élément 106. L'élément structurel interne 106 peut alors s'étendre transversalement à l'intérieur du réservoir 14, de façon à ne pas complètement le diviser en deux parties distinctes, et permettre une circulation d'eau dans le réservoir 14. De façon alternative ou complémentaire, l'élément structurel interne peut pour cela présenter des pores permettant cette circulation.

Selon un exemple illustré par la figure 6B, au moins une électrode 11, 12 peut s'étendre transversalement à l'intérieur du réservoir 14, de façon à le diviser en deux parties distinctes, sans circulation d'eau entre les deux parties. De préférence, une première partie 14a est alors tournée vers, de préférence en contact avec, la face inférieure 101, tandis que la deuxième partie 14b est tournée vers, de préférence en contact avec une face 102 destinée à ne pas être en contact avec la peau. La cathode 12 est de préférence l'électrode faisant cette séparation. Ainsi, l'hydrogène produit dans la première partie 14a est nécessairement acheminé par la face inférieure à la peau, et ne s'échappe pas par une face 102. Les gaz produits dans la deuxième partie 14b, et donc une partie du dihydrogène et le dioxygène, s'échappe par la face 102. La face 102 peut être couverte par un matériau formant une barrière antimicrobienne, comme décrit précédemment.

La face 102 supérieure du dispositif peut ne pas être couverte par un matériau limitant la diffusion du dioxygène, de façon à faciliter l'évacuation du dioxygène hors du corps 10, par une face 102 non en contact avec la peau, comme illustré par exemple par la figure 5. Une face 102 non en contact avec la peau, et de préférence la face 102 supérieure, peut être formée par un matériau présentant un seuil de coupure inférieure à 1 µm, comme décrit précédemment, pour former une barrière antimicrobienne.

Pour les deux exemples décrits ci-dessus, les électrodes 11, 12 sont de préférence disposées dans le réservoir 14. Selon un exemple, les électrodes 11, 12 peuvent être sous la forme de plots, de barre ou de feuillet.

Selon un troisième exemple, illustré par les figures 8A et 8B, uniquement la cathode 12 ou la cathode 12 et l'anode 11 peuvent chacune être formées par une microaiguille, de préférence creuse, destinée à pénétrer dans la peau. L'eau du réservoir 14 peut par exemple être acheminée par capillarité dans l'âme creuse 11a, 12a des électrodes 11, 12 et l'électrolyse peut être effectuée au niveau de leur paroi externe 11b, 12b. L'électrolyse est ainsi effectuée au niveau de la peau, améliorant la délivrance des gaz, et en particulier du dihydrogène, à la peau, tout en favorisant le maintien du corps 10 du dispositif 1 au contact de la peau.

Selon l'un ou l'autre des trois exemples ci-dessus, le corps 10 peut de préférence comprendre plusieurs paires d'une cathode 12 et d'une anode 11, pour former un réseau d'électrodes. Ainsi, la surface de production des gaz par électrolyse est augmentée, pour maximiser la quantité de gaz produite.

Les électrodes peuvent en outre être chacune encapsulées par une membrane semi-perméable entourant chaque électrode, par exemple de type polyéther sulfone, polyamide, polyméthylméthacrylate (PMMA), chitosane, polyvinyle alcool.

Lorsque qu'au moins une électrodes 11, 12 est contenue dans le réservoir 14, la ou les cathodes 12 peuvent être séparées de la ou les anodes 11 par une membrane échangeuse de protons ou membrane à électrolyte polymère (PEM), qui est une membrane semi-perméable fabriquée à partir d'ionomères permettant la conduction protonique tout en étant imperméable aux gaz tels que le dioxygène ou le dihydrogène. Les protons passent à travers alors que les gaz sont stoppés. Cette particularité est exploitée dans les AME (Assemblage de Membrane-Électrode) des piles à combustibles PEM et des électrolyseurs PEM. Les PEM sont fabriquées à partir de membranes en polymère pur ou de membranes composites où les matériaux forment une matrice de polymère. Un des matériaux les plus communément utilisés est le Nafion, un polymère fluoré produit par la société DuPont. La membrane échangeuse de protons peut diviser le réservoir 14 en deux parties. Ainsi, la production de dihydrogène peut être isolée de la production de dioxygène dans le corps 10 du dispositif 1.

La composition des électrodes est adaptée à la fonction de chacune d'elle. Elles peuvent être du même matériau ou de deux matériaux différents. Elles peuvent être à base ou faites de carbone. De préférence, le type de matériau de carbone est choisi parmi le graphite, les nanotubes de carbone, le graphène, du charbon actif ou du diamant. Le matériau des électrodes peut être dopé, notamment au platine, au fer ou à l'or. Les électrodes peuvent être à base ou faites de platine, d'or, d'oxyde d'indium et étain (communément abrégé ITO pour « indium tin oxide »), iridium ou en diamant dopé, en particulier au moins l'anode peut être en or, ou dopée à l'or. Les électrodes peuvent présenter une épaisseur sensiblement comprise entre 100 µm et 2 mm, selon une direction perpendiculaire à la face 101 destinée à être en contact avec la peau, notamment lorsque les électrodes sont sous la forme de plots, de barre ou de feuillet.

Les caractéristiques précédemment décrites sont combinables entre elles (tant qu'elles restent dans le cadre des revendication/s) pour former de nouveaux exemples de réalisation, comme l'illustre par exemple la figure 7.

Nous décrivons dans la suite quelques cas particuliers. Au vu des caractéristiques précédemment décrites, il apparaît par exemple que le dispositif illustré par la figure 2 permet une délivrance à la fois du dihydrogène et du dioxygène à la peau par la face inférieure 101. Le dispositif illustré par les figures 3B ou 3C permet de délivrer uniquement le dihydrogène produit, le dioxygène étant piégé dans le réservoir 14. Les dispositifs illustrés par les figures 4A à 4C permettent une délivrance ciblée de dihydrogène par la ou les ouverture(s) 1010, tandis que le dioxygène reste piégé dans le réservoir 14. Le dispositif illustré par la figure 6A permet une délivrance favorisée du dihydrogène à la peau, une majeure partie du dioxygène étant évacuée par la face 102 supérieure du corps 10.

Au vu de la description qui précède, il apparaît clairement que l'invention propose un dispositif de délivrance de dihydrogène peu invasif, et de préférence non invasif, pour le corps humain et animal, tout en améliorant la délivrance de dihydrogène.

L'invention n'est pas limitée aux modes de réalisations précédemment décrits et s'étend à tous les modes de réalisation couverts par les revendications. La présente invention ne se limite pas aux exemples précédemment décrits. Bien d'autres variantes de réalisation sont possibles, par exemple par combinaison de caractéristiques précédemment décrites, sans sortir du cadre des revendications. En outre, les caractéristiques décrites relativement à un aspect de l'invention peuvent être combinées à un autre aspect de l'invention.

### LISTE DES REFERENCES NUMERIQUES

- 1: Dispositif
- 10: Corps
- 100: Surface externe
- 101: Face inférieure destinée à être en contact avec la peau
- 1010: Ouverture
- 1011: Microaiguille
- 1011a: Âme centrale
- 1011b: Paroi
- 102: Face destinée à ne pas être en contact avec la peau
- 103: Matériau non perméable au dihydrogène
- 104: Matériau présentant un seuil de coupure inférieur à 1 µm
- 105: Matériau semi-perméable au dihydrogène
- 106: Élément structurel interne
- 11: Anode
- 11a: Âme centrale
- 11b: Paroi
- 12: Cathode
- 12a: Âme centrale
- 12b: Paroi
- 13: Source d'énergie électrique
- 14: Réservoir d'eau
- 2: Article vestimentaire
- 3: Corps humain ou animal

## Revendications

1. Dispositif (1) de délivrance de dihydrogène comprenant :
• un corps (10) comprenant au moins une anode (11) et au moins une cathode (12), et
• une source d'énergie électrique (13), l'anode (11) et la cathode (12) étant électriquement raccordées à la source d'énergie électrique (13),
le dispositif (1) étant configuré pour délivrer le dihydrogène à travers la peau d'un corps humain ou animal (3) :
• le corps (10) étant à base d'un matériau souple, apte à se conformer sur la peau d'un corps humain ou animal (3), le corps (10) présentant une surface extérieure (100) comprenant :
∘ une face inférieure (101) destinée à être appliquée sur la peau,
∘ au moins une face (102) destinée à ne pas être en contact avec la peau,
• le corps (10) comprenant un réservoir d'eau (14), l'agencement relatif du réservoir (14), de l'anode (11) et de la cathode (12) étant configuré de sorte que l'eau contenue dans le réservoir est en contact avec l'anode (11) et la cathode (12) pour former un circuit électrique fermé, de façon à produire du dihydrogène à la cathode (12) à partir de l'eau issue du réservoir (14), pour libérer de façon transdermique le dihydrogène produit,
**caractérisé en ce que** le corps (10) est en partie recouvert par un matériau non perméable au dihydrogène (103) formant au moins une portion (1020) de l'au moins une face (102) destinée à ne pas être en contact avec la peau et **en ce que** la face inférieure (101) est au moins en partie recouverte par un matériau semi-perméable (105) présentant une perméabilité supérieure au dihydrogène par rapport à sa perméabilité au dioxygène.

2. Dispositif (1) selon la revendication précédente, dans lequel, le corps (10) est au moins en partie, recouvert par un matériau (104) présentant un seuil de coupure inférieur à 1 µm, formant au moins une portion de l'au moins une face (102) destinée à ne pas être en contact avec la peau.

3. Dispositif (1) selon l'une quelconque des revendications précédentes, dans lequel la face inférieure (101) est au moins en partie recouverte par un matériau semi-perméable (104) présentant un seuil de coupure inférieur à 1 µm.

4. Dispositif (1) selon l'une quelconque des revendications précédentes, dans lequel le matériau semi-perméable (105) présente une perméabilité à l'oxygène inférieure ou égale à 10⁻¹⁷ mol/(m.s.Pa) et une perméabilité à l'hydrogène supérieure ou égale 10⁻¹⁴ mol/(m.s.Pa).

5. Dispositif (1) selon l'une quelconque des revendications précédentes, dans lequel le matériau semi-perméable (105) est perméable au dihydrogène et non perméable au dioxygène.

6. Dispositif (1) selon l'une quelconque des revendications précédentes, dans lequel la face inférieure (101) est uniquement en partie recouverte par un matériau non perméable au dihydrogène (103) de façon à former au moins une ouverture (1010) non recouverte par le matériau non perméable au dihydrogène (103).

7. Dispositif (1) selon la revendication précédente, dans lequel l'au moins une ouverture (1010) est recouverte par un matériau semi-perméable (105) présentant une perméabilité supérieure au dihydrogène par rapport à sa perméabilité au dioxygène, de préférence le matériau semi-perméable (105) est perméable au dihydrogène et non perméable au dioxygène.

8. Dispositif (1) selon l'une quelconque des revendications précédentes, dans lequel la face inférieure (101) est pourvue d'au moins une microaiguille (1011) destinée à pénétrer la peau.

9. Dispositif (1) selon la revendication précédente, dans lequel, la face inférieure (101) étant en partie recouverte par un matériau non perméable au dihydrogène (103) de façon à former au moins une ouverture (1010) non recouverte par le matériau non perméable au dihydrogène (103), l'au moins une microaiguille (1011) est creuse et est disposée en regard de l'ouverture (1010).

10. Dispositif (1) selon l'une quelconque des revendications précédentes, dans lequel le réservoir d'eau (14) comprend un hydrogel.

11. Dispositif (1) selon l'une quelconque des revendications précédentes, dans lequel la cathode (12) et l'anode (11) sont disposées en regard de la face inférieure (101) destinée à être en contact avec la peau.

12. Dispositif (1) selon l'une quelconque des revendications 1 à 10, dans lequel la cathode (12) et l'anode (11) forment un empilement, la cathode (12) étant en regard de la face inférieure (101) destinée à être en contact avec la peau, et l'anode (11) étant en regard d'une face (102) destinée à ne pas être en contact avec la peau.

13. Dispositif selon la revendication précédente, dans lequel l'une parmi la cathode (12) et l'anode (11) s'étendent transversalement dans le réservoir (14) de façon à diviser le réservoir en deux parties distinctes, sans circulation d'eau entre les deux parties, une première partie (14a) communiquant avec la face inférieure (101), une deuxième partie (14b) communiquant avec une face (102) destinée à ne pas être en contact avec la peau.

14. Dispositif (1) selon l'une quelconque des revendications 1 à 10, dans lequel, la face inférieure (101) étant pourvue d'au moins une microaiguille (1011) destinée à pénétrer la peau, la cathode (12) est formée par ladite microaiguille (1011).

15. Article vestimentaire (2) comprenant le dispositif de délivrance (1) de dihydrogène selon l'une quelconque des revendications précédentes.

## Patentansprüche

1. Vorrichtung (1) zur Abgabe von Diwasserstoff, umfassend:
• einen Körper (10), der mindestens eine Anode (11) und mindestens eine Kathode (12) umfasst, und
• eine elektrische Energiequelle (13), wobei die Anode (11) und die Kathode (12) elektrisch mit der elektrischen Energiequelle (13) verbunden sind,
wobei die Vorrichtung (1) so eingerichtet ist, dass sie Diwasserstoff durch die Haut eines menschlichen oder tierischen Körpers (3) abgibt:
• wobei der Körper (10) aus einem weichen Material besteht, das sich an die Haut eines menschlichen oder tierischen Körpers (3) anpassen kann, wobei der Körper (10) eine Außenfläche (100) aufweist, umfassend:
∘ eine Unterseite (101), die dazu bestimmt ist, auf die Haut aufgetragen zu werden,
∘ mindestens eine Seite (102), die dazu bestimmt ist, keinen Hautkontakt zu haben,
• wobei der Körper (10) einen Wasserbehälter (14) umfasst, wobei die relative Anordnung des Behälters (14), der Anode (11) und der Kathode (12) so eingerichtet ist, dass das im Behälter enthaltene Wasser mit der Anode (11) und der Kathode (12) in Kontakt ist, um einen geschlossenen elektrischen Kreislauf zu bilden, um Diwasserstoff an der Kathode (12) aus dem aus dem Behälter (14) stammenden Wasser zu erzeugen, um den produzierten Diwasserstoff transdermal freizusetzen,
**dadurch gekennzeichnet, dass** der Körper (10) teilweise mit einem diwasserstoffundurchlässigen Material (103) bedeckt ist, das mindestens einen Abschnitt (1020) der mindestens einen zur Nichtberührung der Haut bestimmten Seite (102) bildet, und dass die untere Seite (101) mindestens teilweise mit einem halbdurchlässigen Material (105) bedeckt ist, das eine im Verhältnis zu seiner Sauerstoffdurchlässigkeit höhere Diwasserstoffdurchlässigkeit aufweist.

2. Vorrichtung (1) nach dem vorhergehenden Anspruch, wobei der Körper (10) mindestens teilweise mit einem Material (104) bedeckt ist, das eine Abschaltschwelle von weniger als 1 µm aufweist und mindestens einen Abschnitt der mindestens einen Seite (102) bildet, die nicht mit der Haut in Kontakt kommen soll.

3. Vorrichtung (1) nach einem der vorhergehenden Ansprüche, wobei die Unterseite (101) mindestens teilweise mit einem halbdurchlässigen Material (104), das eine Abschaltschwelle von weniger als 1 µm aufweist, bedeckt ist.

4. Vorrichtung (1) nach einer der vorhergehenden Ansprüche, wobei das halbdurchlässige Material (105) eine Sauerstoffdurchlässigkeit von höchstens 10⁻¹⁷ mol/(m.s.Pa) und eine Wasserstoffdurchlässigkeit von mindestens 10⁻¹⁴ mol/(m.s.Pa) aufweist.

5. Vorrichtung (1) nach einem der vorhergehenden Ansprüche, wobei das halbdurchlässige Material (105) diwasserstoffdurchlässig und sauerstoffundurchlässig ist.

6. Vorrichtung (1) nach einem der vorhergehenden Ansprüche, wobei die Unterseite (101) nur teilweise mit einem diwasserstoffundurchlässigen Material (103) bedeckt ist, so dass mindestens eine Öffnung (1010) gebildet wird, die nicht von dem diwasserstoffundurchlässigen Material (103) bedeckt ist.

7. Vorrichtung (1) nach dem vorhergehenden Anspruch, wobei die mindestens eine Öffnung (1010) mit einem halbdurchlässigen Material (105) bedeckt ist, das eine höhere Diwasserstoffdurchlässigkeit im Vergleich zu seiner Sauerstoffdurchlässigkeit aufweist, vorzugsweise ist das halbdurchlässige Material (105) diwasserstoffdurchlässig und sauerstoffundurchlässig.

8. Vorrichtung (1) nach einem der vorhergehenden Ansprüche, wobei die Unterseite (101) mit mindestens einer Mikronadel (1011) zum Eindringen in die Haut versehen ist.

9. Vorrichtung (1) nach dem vorhergehenden Anspruch, wobei die Unterseite (101) teilweise mit einem diwasserstoffundurchlässigen Material (103) bedeckt ist, so dass mindestens eine Öffnung (1010) gebildet wird, die nicht von dem diwasserstoffundurchlässigen Material (103) bedeckt ist, wobei die mindestens eine Mikronadel (1011) hohl ist und gegenüber der Öffnung (1010) angeordnet ist.

10. Vorrichtung (1) nach einem der vorhergehenden Ansprüche, wobei der Wasserbehälter (14) ein Hydrogel umfasst.

11. Vorrichtung (1) nach einem der vorhergehenden Ansprüche, wobei die Kathode (12) und die Anode (11) gegenüber der Unterseite (101) angeordnet sind, die zur Berührung mit der Haut bestimmt ist.

12. Vorrichtung (1) nach einem der Ansprüche 1 bis 10, wobei die Kathode (12) und die Anode (11) einen Stapel bilden, wobei die Kathode (12) gegenüber der Unterseite (101) steht, die zur Berührung mit der Haut bestimmt ist, und die Anode (11) gegenüber einer Seite (102) steht, die nicht zur Berührung mit der Haut bestimmt ist.

13. Vorrichtung nach dem vorhergehenden Anspruch, wobei sich eine der Kathode (12) und die Anode (11) quer in dem Behälter (14) erstrecken, um den Behälter in zwei getrennte Teile zu teilen, ohne Wasserzirkulation zwischen den beiden Teilen, wobei ein erster Teil (14a) mit der Unterseite (101) in Verbindung steht, ein zweiter Teil (14b) mit einer Seite (102) in Verbindung steht, die nicht mit der Haut in Kontakt kommen soll.

14. Vorrichtung (1) nach einem der Ansprüche 1 bis 10, wobei die Unterseite (101) mit mindestens einer Mikronadel (1011) versehen ist, die zum Eindringen in die Haut bestimmt ist, wobei die Kathode (12) von der Mikronadel (1011) gebildet wird.

15. Kleidungsstück (2), das die Vorrichtung (1) zur Abgabe von Diwasserstoff nach einem der vorhergehenden Ansprüche umfasst.

## Claims

1. A dihydrogen delivery device (1) comprising:
• a body (10) comprising at least one anode (11) and at least one cathode (12), and
• an electric energy source (13), the anode (11) and the cathode (12) being electrically connected to the electric energy source (13),
the device (1) being configured to deliver dihydrogen through the skin of a human or animal body (3):
• the body (10) being based on a flexible material, capable of being shaped to the skin of a human or animal body (3), the body (10) having an external surface (100) comprising:
∘ a lower face (101) intended to be applied to the skin,
∘ at least one face (102) intended not to be in contact with the skin,
• the body (10) comprising a water reservoir (14), the relative arrangement of the reservoir (14), the anode (11) and the cathode (12) being configured such that water contained in the reservoir is in contact with the anode (11) and the cathode (12) to form a closed electrical circuit, so as to produce dihydrogen at the cathode (12) from water coming from the reservoir (14), to transdermally release dihydrogen produced,
**characterised in that** the body (10) is partly covered with a dihydrogen impermeable material (103) forming at least one portion (1020) of the at least one face (102) intended not to be in contact with the skin and **in that** the lower face (101) is at least partly covered with a semi-permeable material (105) having a dihydrogen permeability greater than its dioxygen permeability.

2. The device (1) according to the preceding claim, wherein the body (10) is at least partly covered with a material (104) having a cut-off threshold of less than 1 µm, forming at least one portion of the at least one face (102) intended not to be in contact with the skin.

3. The device (1) according to any one of the preceding claims, wherein the lower face (101) is at least partly covered with a semi-permeable material (104) having a cut-off threshold of less than 1 µm.

4. The device (1) according to any one of the preceding claims, wherein the semi-permeable material (105) has an oxygen permeability of less than or equal to 10⁻¹⁷ mol/(m.s.Pa) and a hydrogen permeability of greater than or equal to 10⁻¹⁴ mol/(m.s.Pa).

5. The device (1) according to any one of the preceding claims, wherein the semi-permeable material (105) is permeable to dihydrogen and impermeable to dioxygen.

6. The device (1) according to any one of the preceding claims, wherein the lower face (101) is only partly covered with a dihydrogen impermeable material (103) so as to form at least one opening (1010) not covered by the dihydrogen impermeable material (103).

7. The device (1) according to the preceding claim, wherein the at least one opening (1010) is covered with a semi-permeable material (105) having a dihydrogen permeability greater its dioxygen permeability, preferably the semi-permeable material (105) is permeable to dihydrogen and impermeable to dioxygen.

8. The device (1) according to any one of the preceding claims, wherein the lower face (101) is provided with at least one microneedle (1011) intended to penetrate the skin.

9. The device (1) according to the preceding claim, wherein, the lower face (101) being partly covered with a dihydrogen impermeable material (103) so as to form at least one opening (1010) not covered with the dihydrogen impermeable material (103), the at least one microneedle (1011) is hollow and is disposed facing the opening (1010).

10. The device (1) according to any one of the preceding claims, wherein the water reservoir (14) comprises a hydrogel.

11. The device (1) according to any one of the preceding claims, wherein the cathode (12) and the anode (11) are disposed facing the lower face (101) intended to be in contact with the skin.

12. The device (1) according to any one of claims 1 to 10, wherein the cathode (12) and the anode (11) form a stack, the cathode (12) facing the lower face (101) intended to be in contact with the skin, and the anode (11) facing a face (102) intended not to be in contact with the skin.

13. The device according to the preceding claim, wherein one of the cathode (12) and the anode (11) extends transversely in the reservoir (14) so as to divide the reservoir into two separate parts, without water flowing between the two parts, a first part (14a) communicating with the lower face (101), a second part (14b) communicating with a face (102) intended not to be in contact with the skin.

14. The device (1) according to any one of claims 1 to 10, wherein, the lower face (101) being provided with at least one microneedle (1011) intended to penetrate the skin, the cathode (12) is formed by said microneedle (1011).

15. A cloth item (2) comprising the dihydrogen delivery device (1) according to any one of the preceding claims.
